Europäisches Patentamt

European Patent Office

Office européen des brevets

(11) Publication number: **0 125 354**
**A1**

(12) **EUROPEAN PATENT APPLICATION**

(21) Application number: **83302653.7**

(22) Date of filing: **11.05.83**

(51) Int. Cl.³: **A 61 B 19/00**
**A 61 M 25/02**

(43) Date of publication of application:
**21.11.84 Bulletin 84/47**

(84) Designated Contracting States:
**DE FR GB**

(71) Applicant: **Adair, Edwin L.**
**191 E. Orchard**
**Littleton Colorado 80121(US)**

(72) Inventor: **Adair, Edwin L.**
**191 E. Orchard**
**Littleton Colorado 80121(US)**

(74) Representative: **Corin, Christopher John et al,**
**Mathisen Macara & Co. Lyon House Lyon Road**
**Harrow Middx. HA1 2ET(GB)**

(54) Combined surgical instrument and tube and cord holder device.

(57) A device is provided for yieldably supporting a tube and/or cord extending from a surgical instrument. The device includes a pad (P) which is adhesively securable to a surgical drape or other surface and is connected to a releasable tube-holding means (S) by a stretchable member. The tube-holding means (S) includes a strip having a foam layer (36) on one side and a fabric layer of intertwining material (30) on the other side and a tab (40) attached to one end of the strip and having an interlocked surface which releasably adheres to the fabric layer so as to hold the tube and/or cord in desired location while allowing them to move in response to movement of the surgical instrument. In one embodiment, the outer side of the pad (P) has a layer of intertwining material and a strip of interlacing material is adhesively attached to the surgical instrument so that the instrument can be nested on the pad by pressing the interlacing material against the intertwining material on the pad.

Fig. 2

## COMBINED SURGICAL INSTRUMENT AND TUBE HOLDER DEVICE.

This invention relates to a device for releasably supporting a surgical instrument and a tube or wire connected thereto.

Tube and cord holding devices are well known in the prior art. For example, U.S. Patent Specification No. 3,677, 250 discloses a pair of spaced anchoring pads interconnected by a strap which is adapted to be wrapped around a cord. The pads are each adhesively connected to a supporting surface. This structure has the advantage of holding a tube securely in a predetermined location but permits almost no movement of the tube and therefore is not satisfactory for use at a location close to the surgical device or other instrument which is connected to the tube which must be moved around or manipulated by the user.

Another tube holder is shown in U.S. Patent specification No. 4,074,397 which disloses a pad which is adhesively secured to a supporting surface and has a fabric surface on the other side. A tab is cut from a portion of the pad and has an interlacing surface on the end thereof such as "Velcro" (Registered Trade Mark) so that the tab can be wrapped around the cord and the interlacing means on the end of the tab engaged with the intertwining fabric surface of the pad. As in U.S. patent specification 3,666,250 this device will hold a cord very securely but does not allow movement of this cord upon manipulation of the device to which the cord is attached.

Another tube holder is shown in U.S. patent specification No. 3,782,388 wherein a stretchable cord is connected to a pad which is adhesively adhered to a human body.

- 2 -

The other end of the stretchable cord has an annular clip portion which is removably attached to a cord and allows some movement of the cord on the patient as the patient moves. However, there is no teaching of providing a nesting place on the pad for a surgical instrument when not in use.

The problem to be solved by the present invention is to provide an improved surgical instrument and tube holder device.

The problem is solved by a device characterised by an attachment pad having an adhesive coating on a first side thereof for securing said pad to a fixture, a tube-holding strip having a resilient foam layer on one side and a fabric layer of intertwining material on the opposite side, a tab attached to said strip adjacent one end thereof and extending co-extensively with at least a portion of said foam layer side, said tab having interlacing material on the side thereof facing said foam layer so that when said foam layer is looped around a tube or wire said tab overlaps the fabric side of said strip whereupon said interlacing material is engageable with said intertwining material to releasably hold the tube or wire, and a resilient connecting strap having one end connected to said attachment pad and the other end connected to said tube-holding strip so that the tube or wire is held in a predetermined place but is yieldably movable in response to manipulation of the surgical instrument so as not to restrain movement of the instrument.

It will be understood as used herein that wherever the word "tube" is used that it is intended to include an electrical conduit or wire and wherever the term "surgical instrument" is used it is intended to include any medical device which must be manipulated or adjusted in use and has wires or tubes attached to it.

A device embodying the invention for releasably supporting a surgical instrument will now be described, by way of example, with reference to the accompanying diagrammatic drawing in which;

Figure 1 is a perspective view of a patient operating table or an examination table showing two forms of the tube holder;

Figure 2 is perspective view, to an enlarged scale, showing the use of the attachment pad as a nesting place for a surgical instrument;

Figure 3 is an exploded perspective view of the pad of Figure 2 showing details of the construction thereof;

Figure 4 is a top plan view of the tube-holding strip shown in Figures 1 and 2;

Figure 5 is a side elevation of the tube-holding strap of Figure 4; and

Figure 6 is a side elevation, of the tube-holding strap of Figure 5, but showing a tube in place.

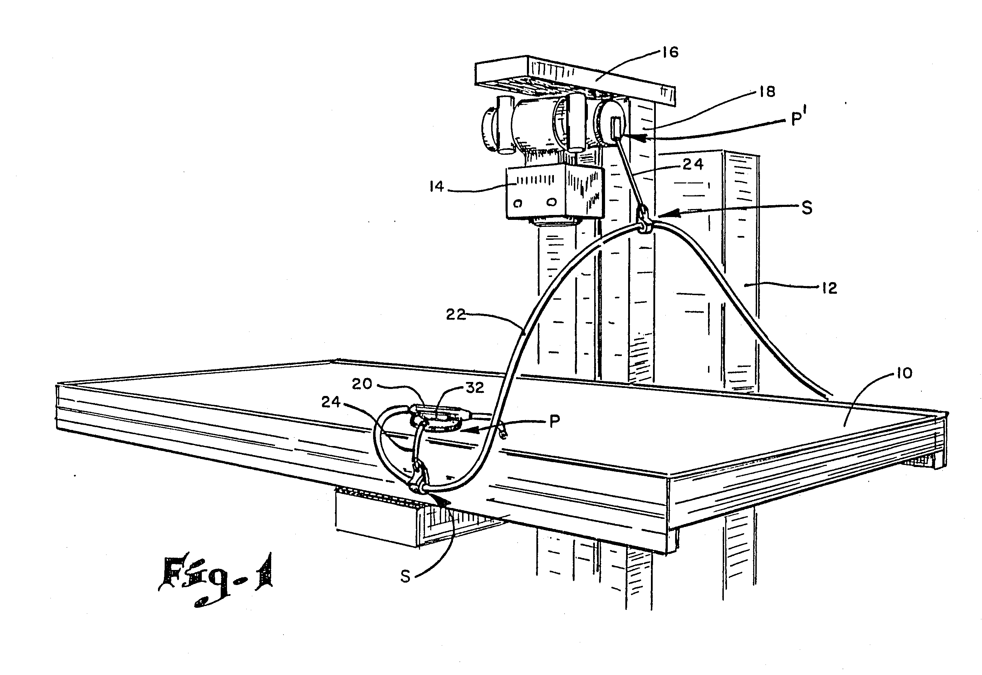

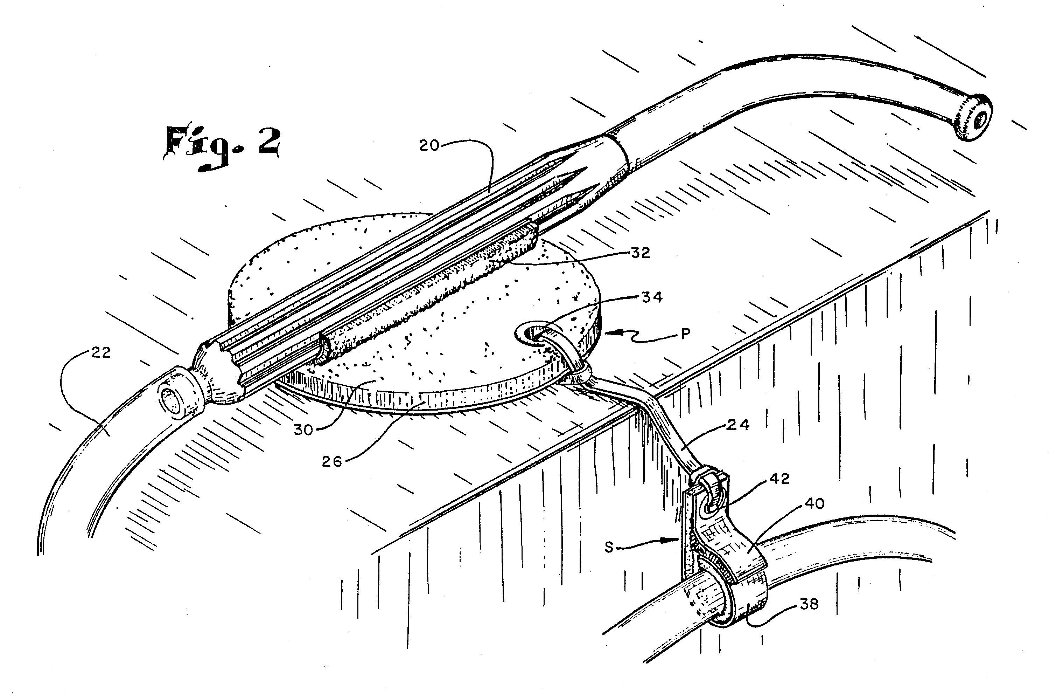

A device for releasably supporting a surgical instrument and a tube or wire connected thereto is provided which as best seen in Figures 1 and 2, can be used with an examination or operating table 10. The table is adjustable up and down on a support 12 and is provided with an X-ray unit 14 supported over the table from an arm 16 attached to a column 18.

A surgical instrument or other medical operative device 20 is connected to a hose 22 and is manipulated by the surgeon, docter, or nurse as required during a surgical or operative procedure. Conveniently, one supporting device is attached to the upper surface of the table or surgical drape by means of a pad P which is interconnected with a tube-holding strip S by means of a resilient strap 24. A second holding device has a pad P' attached to a surface of X-ray unit 14 and is interconnected by a resilient strap 24 with tube-holding strip S, all as seen in Figure 1. It will be understood that the holding device could hold electrical wires as well as tubes and various types of surgical devices or instruments can be used other than the irrigation device illustrated.

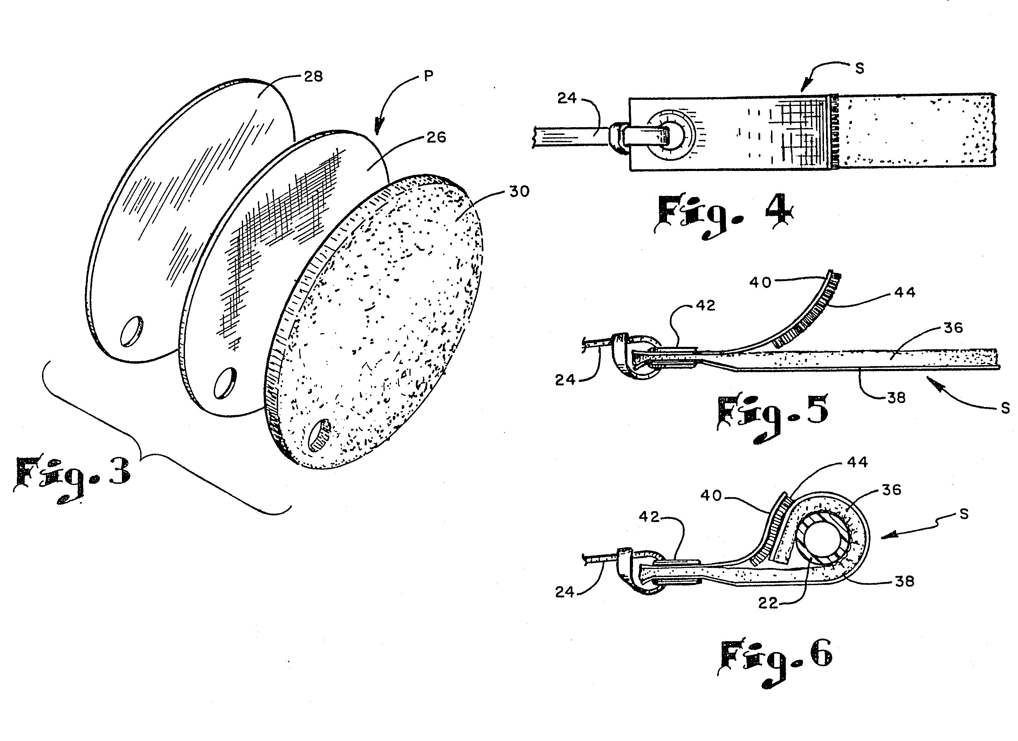

As can best be seen in Figure 3, pad P comprises a fabric layer 26 having an adhesive surface on one side thereof for adhesively applying the pad to the table or surgical drape. Conveniently, layer 26 is protected by a removable cover 28 prior to use. The other side of fabric layer 26 is permanently attached to a nesting surface 30 having a relatively loosely woven intertwining material thereon. Conveniently, instrument 20 has an attachment strip 32 adhesively attached thereto and having a surface of interlacing material with a plurality of small hooks, such as "Velcro" (Registered Trade Mark) which releasably interlocks with the intertwining surface of the pad to provide a nesting place for the instrument when not in use.

Pad P also is provided with an eyelet 34 through which a resilient strap 24 is attached as shown. This strap may be made of any resilient material but in practice a rubber band has been found to be entirely satisfactory.

Conveniently, the other end of strap 24 is connected to holding-strip S whose structure can best be seen in Figures 2, 4, 5 and 6. Strip S includes a length of foam material 36 conveniently attached to a layer of supporting material 38, such as a piece of fabric. A tab comprising a second strip of fabric 40 is connected to one end of the foam material, as by an eyelet 42, the other end of strip 40 being provided with a layer of interlacing material 44, such as "Velcro". As best seen in Figures 2 and 6, the foam material 36 can be wrapped around tube 22 and interlacing material 44 on the tab 40 brought into contact with the fabric supporting material surface 38 to hold the tube in place. Thus, the holding device shown serves a dual purpose of providing a nesting or holding place for the instrument when not in place on top of the pad and also provides a resilient holding means for the tube providing some movement for the tube when the instrument is being manipulated by the user. A second embodiment is shown in Figure 1 wherein pad P' is of generally rectangular configuration, although the particular shape is unimportant, which is attached to the X-ray unit and supports a tube but does not serve as a nesting place for a surgical instrument.

From the foregoing, the advantages of the preferred embodiment are readily apparent. A surgical instrument and tube-holding device has been provided which has the function in one embodiment of releasibly holding a tube in a relatively fixed position but allowing it to be moved in response to manipulation of the instrument by the user. Another embodiment provides a dual function of not only holding a tube but also providing a nesting place for the instrument when it is not in use.

## CLAIMS.

1.   A device for releasably supporting a surgical instrument and a tube or wire connected thereto, said device being characterised by an attachment pad (P) having an adhesive coating (26) on a first side thereof for securing said pad to a fixture (10), a tube-holding strip (S) having a resilient foam layer (36) on one side and a fabric layer of intertwining material (30) on the opposite side, a tab (40) attached to said strip (S) adjacent one end thereof and extending co-extensively with at least a portion of said foam layer side, said tab having interlacing material (44) on the side thereof facing said foam layer so that when said foam layer is looped around a tube or wire said tab overlaps the fabric side of said strip (S) whereupon said interlacing material is engageable with said intertwining material (30) to releasably hold the tube or wire, and a resilient connecting strap (24) having one end connected to said attachment pad (P) and the other end connected to said tube-holding strip (S) so that the tube or wire is held in a predetermined place but is yieldably movable in response to manipulation of the surgical instrument so as not to restrain movement of the instrument.

2.   A device as claimed in claim 1, further characterised by a first eyelet (34) in said pad and a second eyelet (42) in said one end of said tube-holding strip (40), said one end of said connecting strap (24) being connected to said first eyelet (34) and said other end of said strap (24) being connected to said second eyelet (42).

3. A device as claimed in claim 2, characterised in that said first eyelet (34) is adjacent the edge of said pad (P).

4. A device as claimed in claim 2 or claim 3 characterised in that said second eyelet (42) attaches said tab (40) to said strip (S).

5. A device as claimed in any one of claims 1 to 4 characterised in that said strap (24) is a rubber band.

6. A device as claimed in any one of claims 1 to 5 characterised in that said attachment pad has an intertwining surface (32) on its opposite second, side.

7. A device as claimed in any one of claims 1 to 6 characterised by an attachment strip (32) having an adhesive coating on one side for attaching it to a surgical instrument and a surface of interlacing material on the other side for engagement with said intertwining surface (30) of said pad (P) to provide a nesting place for the instrument when not in use.

Fig-1

0125354

Fig. 2

28

P

26

30

Fig. 3

24

S

Fig. 4

40

42

44

36

24

38

S

Fig. 5

44

40

36

42

S

24

38

22

Fig. 6

3/3

0125354

European Patent Office

**EUROPEAN SEARCH REPORT**

Application number

EP 83 30 2653

## DOCUMENTS CONSIDERED TO BE RELEVANT

| Category | Citation of document with indication, where appropriate, of relevant passages | Relevant to claim | CLASSIFICATION OF THE APPLICATION (Int. Cl. ³) |
|---|---|---|---|
| Y | US-A-3 288 136 (LUND)<br>* Figures 1-5; claim 1; column 1, lines 33-42 * | 1,6,7 | A 61 B 19/00<br>A 61 M 25/02 |
| Y | US-A-4 088 136 (HASSLINGER)<br>* Figures 2,5; column 7, lines 18-29; column 3, lines 12-52 * | 1,6,7 | |
| A | US-A-2 669 231 (FISHER)<br>* Figures * | 2-5 | |

TECHNICAL FIELDS SEARCHED (Int. Cl. ³)

A 61 B
A 61 M
A 61 G

The present search report has been drawn up for all claims

| Place of search | Date of completion of the search | Examiner |
|---|---|---|
| THE HAGUE | 17-01-1984 | LOWE D. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503. 03 82